# EUROPEAN PATENT SPECIFICATION

(11) **EP 0 448 969 B1**
(45) Date of publication and mention of the grant of the patent: **15.05.1996**
(21) Application number: 91102793.6
(22) Date of filing: 26.02.1991
(51) Int. Cl.: C12N 15/53, C12N 9/02

(54) **Structural gene of membrane-bound alcohol dehydrogenase complex,plasmid containing the same and transformed acetic acid bacteria**
Strukturgen von membrangebundenem Alkoholdehydrogenase-Komplex, Plasmid, das dieses Gen enthält und transformierte Essigsäurebakterien
Gène de structure de complexe d'alcool déhydrogénase lié à une membrane, plasmide contenant le gène et bactéries transformées d'acide acétique

(30) Priority: 26.03.1990 JP 42391/90; 26.03.1990 JP 73440/90
(43) Date of publication of application: 02.10.1991
(73) Proprietor: NAKANO VINEGAR CO., LTD., Handa-shi, Aichi-ken 475 (JP)
(72) Inventor: Tamaki, Toshimi, Aiichi-ken, 475 (JP); Takemura, Hiroshi, Toda-shi, Saitama-ken, 335 (JP); Tayama, Kenji, Handa-shi, Aichi-ken 475 (JP); Fukaya, Masahiro, Chita-gun, Aichi-ken 470-21 (JP); Okumura, Hajime, Aichi-ken, 475 (JP); kawamura, Yoshiya, Kounan-shi, Aichi-ken, 483 (JP)
(74) Representative: VOSSIUS & PARTNER

(56) References cited:
- PATENT ABSTRACTS OF JAPAN, unexamined applications, C field, vol. 14, no. 126, 09 March 1990, The Patent Office Japanese Govt.; p. 4 C 699
- JOURNAL OF BACTERIOLOGY, vol. 171, no. 6, June 1989, Baltimore, MD (US); T. INOUE et al., pp. 3115-3122

## Description

The present invention relates to a structural gene of membrane-bound alcohol dehydrogenase complex derived from a microorganism belonging to the genus Acetobacter, and a plasmid containing the same as well as its utilization.

A membrane-bound alcohol dehydrogenase produced by a microorganism belonging to the genus Acetobacter is an enzyme which oxidizes an alcohol into the corresponding acid . The enzyme takes a part in oxidative fermentation of acetic acid fermentation for producing acetic acid from ethanol, and is also utilized for quantitative determination of alcohol; the enzyme is useful from an industrial viewpoint.

Heretofore the membrane-bound alcohol dehydrogenase (hereafter simply referred to as ADH) has been obtained by culturing a microorganism belonging to the genus Acetobacter or the genus Gluconobacter, extracting and purifying from the cultured cells and has been utilized (Agricultural and Biological Chemistry, 42, 2045, 1978; ibid., 42, 2331, 1978).

For purification of this enzyme, however, fractionation by complicated column chromatography was required so that it was difficult to prepare the enzyme in large quantities. In addition, the enzyme is unstable and cannot be stored over a long period of time, which has been a problem in its application.

In order to solve these problems, it is considered to harvest mutants having an enhanced enzyme content in the cells by a mutation treatment. However, there is no report yet that any mutant having a sufficient enzyme content was harvested. It is also considered to achieve the object by cloning a gene of the enzyme and increasing the copy number of the enzyme gene or enhancing an expression degree, through genetic engineering technology. For this attempt, ADH gene of Acetobacter aceti K6033 strain has been cloned and its nucleotide sequence has been determined (Journal of Bacteriology, 171_{,} 3115, 1989). This study is expected to be effective for improving the productivity of the enzyme by genetic engineering technology. In actuality, however, even though a plasmid carrying the enzyme gene is introduced into a host of acetic acid bacteria, the enzyme activity is not improved more than the activity inherently possessed by the host and such technique is not practical.

This is believed to be because the cloned gene would be composed only of subunits having a larger molecular weight out of the subunits constructing ADH. Any conventional ADH is purified in the form of a complex with cytochrome c from acetic acid bacteria. Matsushita et al. reported that the activity of ADH was affected depending upon the quantity of cytochrome c and cytochrome c was not present merely as an impurity but took a part in expressing the enzyme activity (Agricultural and Biological Chemistry, 53, 2895, 1989). For this reason, it was necessary to increase the subunits having a large molecular weight and at the same time, increase the amount of the subunits of cytochrome c.

Furthermore, properties of the enzyme in the cloned Acetobacter aceti K6033 strain were not studied and utility of the enzyme of K6033 strain is unclear.

In order to solve the foregoing problems, the present inventors have brought attention to ADH produced by a series of microorganisms belonging to the genus Acetobacter represented by Acetobacter altoacetigenes, which are already known to have enzymatically excellent properties, and have succeeded in cloning the structural gene of two proteins (subunits) constructing ADH and in carrying the structural gene on a plasmid.

Furthermore, the present inventors have found that by using the plasmid carrying the cloned gene, the content of this enzyme in the cells can be increased, ADH can be readily extracted and purified, and the efficiency of acetic acid fermentation can be improved. The present invention has thus been accomplished.

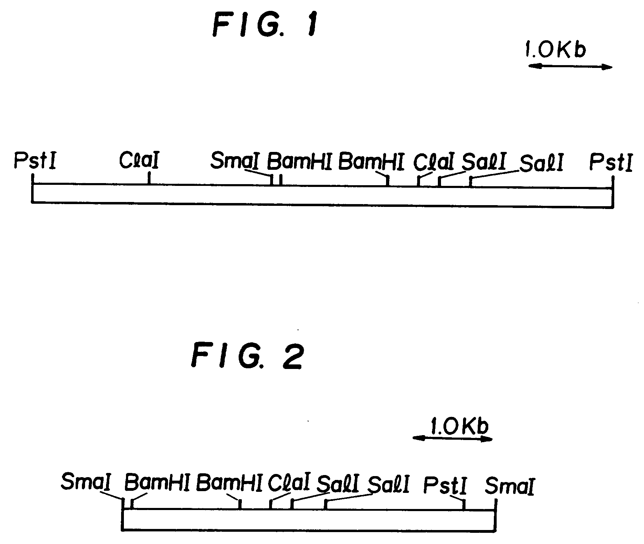

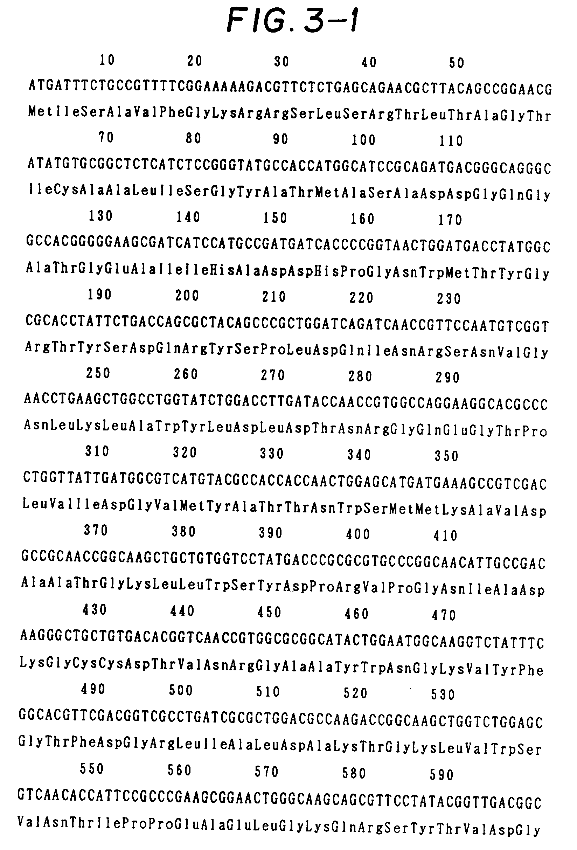

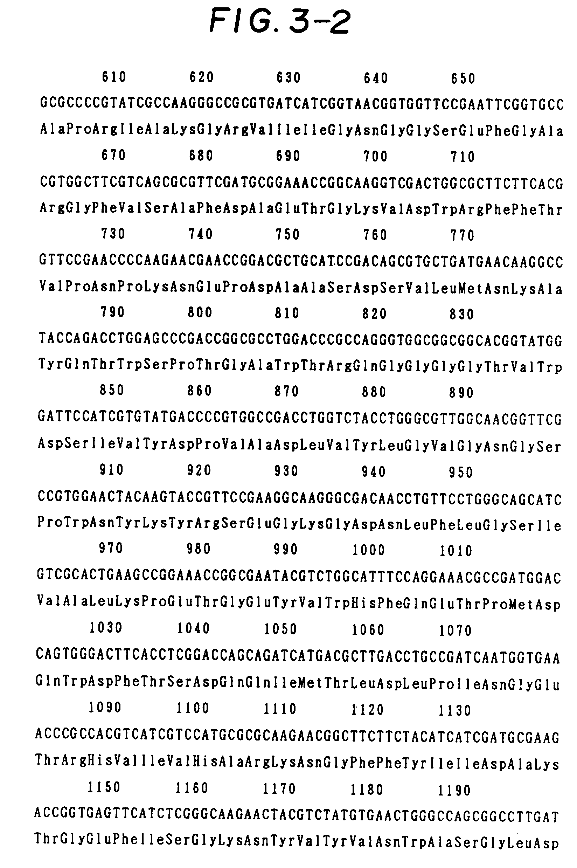

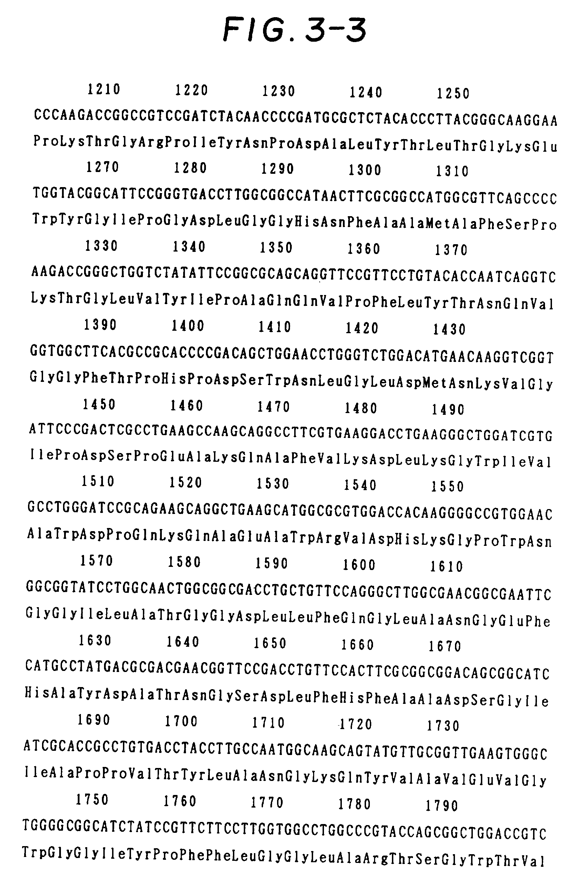

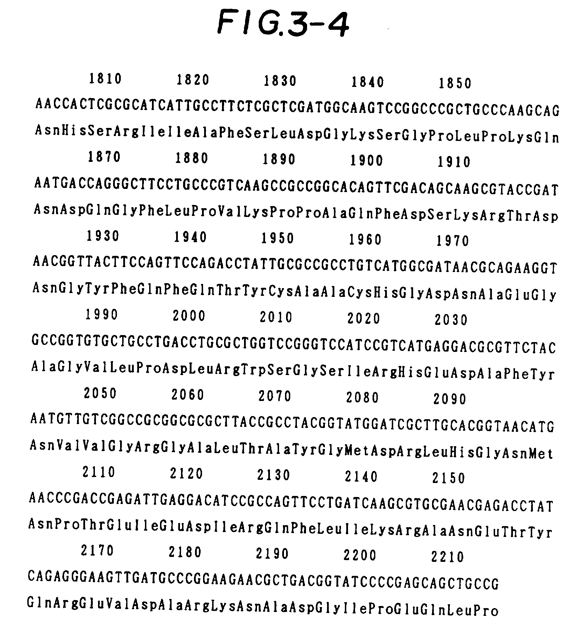

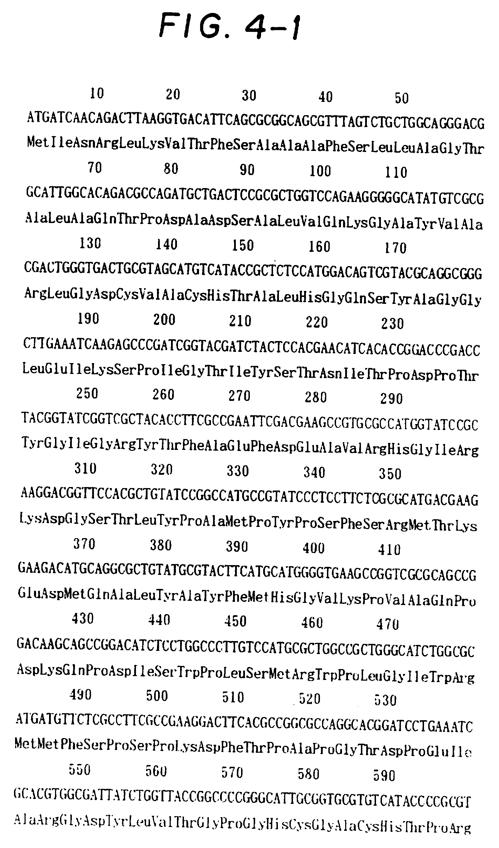

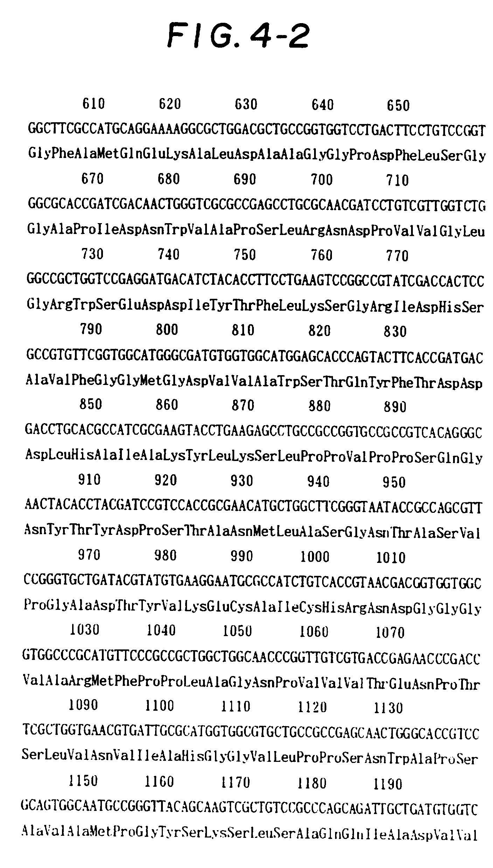

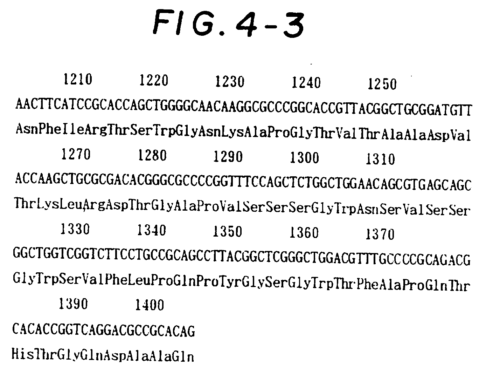

The present invention relates to a structural gene of ADH complex which is derived from a microorganism belonging to the genus Acetobacter, has the restriction map given in Fig. 1 and has a length of about 7.0 kilobase pairs (kb). The invention further relates to a plasmid carrying the gene as well as a microorganism belonging to the genus Acetobacter or the genus Gluconobacter transformed with the plasmid. The present invention further relates to a structural gene of a protein constituting an ADH complex, which is represented by the nucleotide sequence shown in Fig. 3 and has a molecular weight of about 72,000, and a plasmid carrying the gene as well as a microorganism belonging to the genus Acetobacter or the genus Gluconobacter transformed with the plasmid. The present invention also relates to a structural gene of a protein constituting an ADH complex, which is represented by the nucleotide sequence shown in Fig. 4 and has a molecular weight of about 44,000, and a plasmid carrying the gene as well as a microorganism belonging to the genus Acetobacter or the genus Gluconobacter transformed with the plasmid.

Fig. 1 shows the restriction map of the structural gene of a membrane-bound alcohol dehydrogenase complex isolated using Pst I.

Fig. 2 shows the restriction enzyme map of the structural gene of a protein having a molecular weight of about 72,000 which constitutes a membrane-bound alcohol dehydrogenase complex isolated using Sma I.

Fig. 3: the upper lines show the nucleotide sequence of the structural gene of a protein having a molecular weight of about 72,000 which constitutes a membrane-bound alcohol dehydrogenase complex isolated using Sma I, and the lower lines show the amino acid sequence determined from the nucleotide sequence of the structural gene of a membrane-bound alcohol dehydrogenase complex.

Fig. 4: the upper lines show the nucleotide sequence of the structural gene of a protein having a molecular weight of about 44,000 which constitutes a membrane-bound alcohol dehydrogenase complex isolated using Pst I, and the lower lines show the amino acid sequence determined from the nucleotide sequence of the structural gene of a membrane-bound alcohol dehydrogenase complex. Abbreviations in the amino acid sequences have the following meaning:

| | | | |
|---|---|---|---|
| Met | methionine | Ala | alanine |
| Arg | arginine | Asn | asparagine |
| Asp | aspartic acid | Cys | cystein |
| Gln | glutamine | Glu | glutamic acid |
| Gly | glycine | His | histidine |
| Ile | isoleucine | Leu | leucine |
| Lys | lysine | Phe | phenylalanine |
| Pro | proline | Ser | serine |
| Thr | threonine | Trp | tryptophan |
| Tyr | tyrosine | Val | valine |

The membrane-bound alcohol dehydrogenase complex in the present invention refers to a novel alcohol dehydrogenase complex having excellent stability which is described in Japanese Patent Application Laid-Open No. 63-12278 and composed of two proteins having molecular weights of about 72,000 and about 44,000. This enzyme is produced by a series of microorganisms belonging to the genus Acetobacter represented by Acetobacter altoacetigenes MH-24 (FERM BP-491).

The gene fragment containing the structural gene of the enzyme can be cloned from the total DNA which the microorganism belonging to the genus Acetobacter capable of producing this enzyme carries. The total DNA may be prepared by, for example, the method disclosed in Japanese Patent Application Laid-Open No. 60-9489. The gene fragment containing the structural gene of the ADH complex may be cloned from the total DNA by, for example, the procedures shown in Example 1, that is, determining a part of the amino acid sequence of this enzyme, preparing synthetic DNA corresponding to the determined amino acid sequence and selecting a clone having the desired gene utilizing the synthetic DNA as a probe; etc. The amino acid sequence may be determined as follows: after the alcohol dehydrogenase complex purified by the method disclosed in Japanese Patent Application Laid-Open No. 63-12278 is separated into two subunits by SDS-polyacrylamide gel electrophoresis, the protein corresponding to each subunit is extracted from the gel in a conventional manner such as electric dialysis, etc. The extracted protein is used for determination of amino acid sequence at the amino terminus as it is. Alternatively, after the protein is cleaved with CNBr or with a protease (peptidase) having a high specificity, the cleavage product is fractionated by gel filtration, etc. and the resulting fraction is used for determination of amino acid sequence at the amino terminus in a conventional manner using an amino acid sequencer, etc. Synthesis of DNA corresponding to the thus determined amino acid sequence may be carried out in a conventional manner.

An antibody to the enzyme may be prepared by separating into two subunits the alcohol dehydrogenase complex purified by the method disclosed in Japanese Patent Application Laid-Open No. 63-12278 by SDS-polyacrylamide gel electrophoresis, extracting the protein corresponding to each subunit from the gel in a conventional manner such as electric dialysis, etc. and using the extracted protein as an antigen. Specifically, anti-ADH antibody may be obtained by, for example, the method described in "Methods in Enzymology", 73, 46 (1981). About 2 weeks after the first immunization, a second immunization is made and in a month to a month and a half, the production of the antibody specific to ADH is observed. This antibody may be further purified either through purification by ammonium sulfate fractionation, etc. or by ion exchange chromatography. In the case that the antibody is used to clone the gene, it may also be possible to use appropriately diluted serum.

On the other hand, the cleavage product of the total DNA with an appropriate restriction enzyme is ligated with the cleavage product of an appropriate vector with a restriction enzyme capable of ligating with the total DNA using T4 DNA ligase. The ligation product is transformed to E. coli host. Examples of the vector used in this case include vectors of E. coli generally used, such as pBR322, pUC18, pUC19, and the like.

Transformation of E. coli may be conducted in a conventional manner. Detection of a strain bearing the desired gene can be made by preparing synthetic DNA based on the amino acid sequence previously determined using the purified enzyme and performing conventional colony hybridization using the synthetic DNA as a probe, whereby a strain reactive with the probe is selected.

Also where antigen-antibody reaction is utilized, a strain carrying the desired gene may be detected by a method similar to, e.g., Gene, 37, 267 (1985). That is, the lysate of the resulting transformants is reacted with the antibody and a strain showing a specific reaction may be selected.

The strain selected by the procedures described above may have a plasmid carrying the gene fragment having the entire length of the desired gene but may sometimes carry merely a part of the gene.

Where the strain has merely a part of the gene, the entire length of the gene may be obtained by using as a probe the gene already obtained and isolating a fraction showing homology to the probe by Southern hybridization, etc.

The nucleotide sequence of the resulting gene may be determined in a conventional manner, for example, by the dideoxy method using M13 phage.

In order to produce the ADH complex or the proteins constructing the ADH complex using the thus isolated gene fragment containing the structural gene of the ADH complex, in general, it is necessary to ligate the gene fragment carrying the enzyme gene with a gene having a promoter activity functioning in a host in the form of capable of expression. As the promoter used to produce the ADH complex proteins in a microorganism belonging to the genus Acetobacter or the genus Gluconobacter, there may be used a promoter inherently possessed by the ADH complex gene and there may also be used an acetic acid bacteria-derived gene having other promoter activity and a promoter of E. coli capable of expressing in acetic acid bacteria. As the E. coli promoter, there may be used promoters of ampicillin-resistant gene of E. coli plasmid pBR322, kanamycin-resistant gene of E. coli plasmid pACYC177, chloramphenicol-resistant gene of E. coli plasmid pACYC184, B-galactosidase gene of E. coli, etc. Where the ADH complex is produced in an excess amount to affect growth or the like of the host, it is necessary to choose an appropriate promoter for controlling an expression amount of the gene. Where the gene is expressed, formation of a protein having a size different from the molecular weight of the gene is sometimes observed. This is because the protein is produced in a host in the form of a fused protein in which other protein is fused. However, if the fused protein is produced in such a form that its enzyme activity can be expressed, there would be no problem.

As the vector for carrying the gene fragment containing the structural gene of the ADH complex in acetic acid bacteria, there may be utilized, for example, pTA5001(A) and pTA5001(B) disclosed in Japanese Patent Application Laid-Open No. 60-9488; wide host range vectors RP4::Mu, RP4, pRK2013, RSF1010 etc. which can be introduced into acetic acid bacteria.

For expression of the activity of ADH, it is necessary that the two proteins constituting the ADH complex be produced efficiently with good balance, as shown in the EXAMPLES. In general, the gene fragment containing the structural gene of the ADH complex is used as it is and the two proteins may be expressed on the same level. Depending upon acetic acid bacteria, however, either protein is not sufficiently possessed in some occasion. In this case, it is required that the gene encoding the two proteins are independently prepared and the genes having a promoter activity used to express the genes are selected to be a suitable expression amount, respectively. For controlling the expression amount, it may also be possible to use different vectors in the two genes and utilize a difference in the copy number of the vectors in acetic acid bacteria.

As stated above, the plasmid containing the structural gene of the ADH complex can be isolated. After transformation, the gene is expressed, whereby the protein constituting the ADH complex can be produced in a marked quantity.

As the host for producing the ADH complex, microorganisms such as E. coli, Bacillus subtilis, etc. on which genetic engineering technique has been established may be used. However, it is more advantageous to use acetic acid bacteria which inherently possess the ability of producing the ADH complex, namely, the microorganisms belonging to the genus Acetobacter or the genus Gluconobacter.

ADH has pyrroloquinoline quinone (PQQ) as its prosthetic group. In order to produce an activated enzyme, PQQ may be supplemented to a medium, etc. to produce the ADH complex protein. However, as is described in Agricultural & Biological Chemistry, 48, 561 (1984), the ability of E. coli or S. subtilis for synthesizing PQQ is poor and it has been revealed that the synthesizing ability of acetic acid bacteria is high. It is thus advantageous for the host to have the ability for synthesizing PQQ.

Further in acetic acid fermentation, ADH participates in the reaction of oxidizing ethanol to acetaldehyde. For this reason, by enhancing the content of the ADH complex in acetic acid bacteria, it can be expected to make the acetic acid fermentation efficient. In this case, where ADH alone is expressed excessively, the concentration of acetaldehyde, which is the oxidation product of ethanol, increases so that acetic acid bacteria are damaged by strongly cytotoxic acetaldehyde. Therefore, it is necessary either to control the amount of the ADH complex gene expressed to the amount corresponding to the oxidising activity of acetaldehyde or to increase the amount of aldehyde dehydrogenase at the same time, using the structural gene of the membrane-bound aldehyde dehydrogenase recited in Japanese Patent Application Laid-Open No. 63-52709 so as not to cause excessive accumulation of acetaldehyde.

### [EXAMPLES]

The present invention is illustrated by the following examples.

### EXAMPLE 1

### [Determination of amino terminal amino acid sequence and preparation of synthetic probe]

Acetobacter altoacetigenes MH-24 (FERM BP-491) strain was shaking cultured at 30°C in medium composed with 3% of glucose, 4% (V/V) of ethanol, 6% (V/V) of acetic acid, 0.5% of yeast extract (manufactured by Daigo Nutrient Chemistry Co., Ltd.) and 0.2% of polypeptone (manufactued by Daigo Nutrient Chemistry Co., Ltd.).

After the incubation, the cells were harvested by centrifugation and 10 mg of the ADH complex was then obtained in a conventional manner (the method disclosed in Japanese Patent Application Laid-Open No. 63-12278). This complex was subjected to SDS-polyacrylamide gel electrophoresis to separate the protein having a molecular weight of about 72,000 and the protein having a molecular weight of about 44,000. Then, the protein of 72,000 was eluted from the gel in a conventional manner and provided as a sample for the following experiment.

After 1 mg of the sample obtained was cleaved with lysyl endopeptidase (manufactured by Wako Pure Chemicals, Inc.), the cleavage product was fractionated by HPLC LC-4A manufactured by Shimadzu Seisakusho Co., Ltd. As a column Senshu Pak. VP-304-1251 (4.6 ø x 250 mm) was used and the elution was performed at a flow rate of 1 ml/min and at room temperature by linear gradient of acetonitrile-water (containing 0.1% trifluoroacetic acid) of 0 to 55%. By monitoring at absorbance of 220 nm, 11 peaks were noted. From the earlier order of elution, the second, ninth and eleventh peaks were fractionated. About 0.5 mg of the fractionated product was applied to amino acid sequencer Model 470A manufactured by Applied Biosystems Inc. to determine the amino terminal amino acid sequence. The results reveal that the sequence of the peptide eluted in the ninth order was:
Thr-Gly-Leu-Val-Tyr-Ile-Pro-Ala-Gln-Gln-Val-Pro-Phe-Leu-Tyr-Thr-Asn-Gln-Val-Gly-Gly-Phe-Tyr-Pro-His-Pro-Asp; and that the sequence of the peptide eluted in the eleventh order was: Leu-Ala-Trp-Tyr-Leu-Asp-Leu-Asp-Thr-Asn-Arg-Gly-Gln-Glu-Gly-Thr-Pro-Leu. Furthermore, the sequence of the peptide eluted in the second order was: Asn-Tyr-Val-Tyr-Val-Asn-Trp-Ala-Ser-Gly-Leu-Asp-Pro.

The protein having a molecular weight of 72,000 which was not treated with lysyl endopeptidase was analyzed with an amino acid sequencer. The amino terminal amino acid sequence was Asp-Asp-Gly-Gln-Gly. DNA corresponding to the amino acid sequence was synthesized with DNA synthesizer 381A manufactured by Applied Biosystems Inc., based on the two sequences Tyr-Ile-Pro-Ala-Gln-Gln-Val (Sequence 1) and Val-Ile-Ile-Gly-Asn-Gly (Sequence 2) in the amino acid sequence of the peptide eluted in the ninth order and a part of the amino acid sequence, Try-Val-Tyr-Val-Asn-Trp-Ala (sequence 3), in the peptide eluted in the second order, taking utilization of codon into account.

For Sequence 1, Probe 1 a 64-fold degenerate 20-mer was synthesized:

  T  T
TA AT CCNGCNCAGCAGCAGG
  C  C

For Sequence 2, Probe 2, a 128-fold degenerate 17-mer was synthesized:

      T  T     T
GTNAT AT GGNAA GG
      C  C     C

For Sequence 3, Probe 3, a 128-fold degenerate 20-mer was synthesized:

      A     A  A A
GCCCA TTNAC TAN C TA
      G     G  G G

This sequence was derived from the complementary strand.

### Cloning of the structural gene of Protein having a molecular weight of about 72.000 which constructs the ADH complex

From the cells of Acetobacter altoacetigenes MH-24 strain which had been obtained by culturing as described above, the total DNA was prepared in a conventional manner (the method disclosed in Japanese Patent Application Laid-Open No. 60-9489). After the total DNA was cleavedwith arestriction enzyme, Pst I or Sma I (manufactured by Takara Shuzo Co., Ltd.), the product was ligated with E. coli vector pUC18 (manufactured by Takara Shuzo Co., Ltd.) which was cleaved with Pst I or Sma I, and thereafter dephosphorylated with bacterial alkaline phosphatase (manufactured by Takara Shuzo Co., Ltd.), using T4 DNA ligase (manufactured by Takara Shuzo Co., Ltd.). After the ligation mixture was transformed to the host E. coli JM 109 by the method of Hanahan ["DNA Cloning", 1, 109, IRL Press (1985)], transformants were selected in LB agar medium ("A Manual for Genetic Engineering", page 201, Cold Spring Harbor Laboratory, 1980) containing ampicillin in a concentration of 30 µg/ml.

With respect to about 5,000 recombinants obtained, colonies which hybridized with Probe 2 and Probe 3 described above were detected according to the colony hybridization technique ("A Manual for Genetic Engineering", page 312, Cold Spring Harbor Laboratory, 1980) using the two probes. In Pst I, three (3) clones hybridized with Probes 2 and 3,and in Sma I, two (2) clones hybridized with the probes. Furthermore, these 5 clones all hybridized also with Probe 1.

Analysis with restriction enzyme reveals that all of the 3 clones obtained using Pst I had the same fragment of about 7.0 kilo base at the Pst I site of pUC18. Further,in the case of Sma I, the clones had the same fragment of about 4.5 kilo base. The fragment of about 7.0 kilo base obtained with Pst I had a portion of about 4.1 kilo base in common to the fragment of about 4.5 kilo base. The plasmid (chimeric plasmid composed of pUC18 and the insert fragment of about 7.0 kilo base, named pADHP1) possessed by 1 clone obtained using Pst I was transformed in E. coli JM 109 and has been deposited in the Fermentation Research Institute of the Agency of Industrial Science and Technology of Japan under the name of E. coli ADHP-1 as [FERM BP-3254 (FERM P-11278)]. The restriction enzyme map of the insert fragment of about 7.0 kilo base was prepared in a conventional manner, which is as shown in Fig. 1. Furthermore, the plasmid (chimeric plasmid composed of pUC18 and the insert fragment of about 4.5 kilo base, named pADHS1) possessed by 1 clone obtained using Sma I was transformed in E. coli JM 109 and has been deposited in the Fermentation Research Institute of the Agency of Industrial Science and Technology of Japan under the name of E. coli ADHS-1 as [FERM BP-3253 (FERM P-11201)]. The restriction enzyme map of the insert fragment of about 4.5 kilo base was prepared in a conventional manner, which is as shown in Fig. 2.

With respect to the insert fragment of pADHS1, its nucleotide sequence was determined by the dideoxy method [Methods in Enzymology, 10, 20, Academic Press, New York, 1983] using M13 phage.

Based on the thus determined nucleotide sequence, an open-reading frame was identified. The open-reading frame encoding 738 amino acid residues (molecular weight 80839), composed of 2214 bases and translated from the ATG initiation codon as shown in Fig. 3 was identified in the portion common to the Sma I fragment having a size of about 4.5 kilo base and the Pst I fragment having a size of about 7.0 kilo base (the amino acid sequence determined from the nucleotide sequence of Fig. 3 is shown in Fig. 3, lines below the nucleotide sequence). The polypeptide encoded by the nucleotide sequence of Fig. 3 coincides with the protein having a molecular weight of about 72,000 which constitutes the membrane-bound alcohol dehydrogenase complex of the present invention. This is confirmed by the fact that when the amino acid sequence of the purified protein having a molecular weight of about 72,000, which constructs the membrane-bound alcohol dehydrogenase complex of the present invention, was determined by the method described above, the sequence fully coincident with the amino terminal amino acid sequences of the 3 peptides of the lysyl endopeptidase cleavage products was found. That is, the sequence of 27 amino acids of the peptide eluted in the ninth order coincided with the sequence of 27 amino acids following 442 amino acid from the amino terminus deduced from the nucleotide sequence. Furthermore, the sequence of 18 amino acids of the peptide eluted in the eleventh order coincided with the sequence of 18 amino acids following 84 amino acid deduced from the nucleotide sequence. The amino terminal amino acid sequence of the peptide eluted in the second order coincided with the sequence of 13 amino acids following 389 amino acid deduced from the nucleotide sequence.

Furthermore, the amino terminal sequence (Asp-Asp-Gly-Gln-Gly) of the purified protein completely coincided with the amino acid sequence following the 36th counted from the amino terminus which is deduced from the nucleotide sequence. It is thus assumed that the amino acid sequence up to the 35th from the amino terminus deduced from the nucleotide sequence would be the region which participates in secretion of the protein having a molecular weight of about 72,000. Acetobacter aceti K6033 strain had homology of about 77% to ADH gene in the amino acid sequence.

### Preparation of anti-ADH antibody

Acetobacter altoacetigenes MH-24 (FERM BP-491) strain was shakingly cultured at 30°C in medium composed with 3% of glucose, 4% (V/V) of ethanol, 6% (V/V) of acetic acid, 0.5% of yeast extract (manufactured by Daigo Nutrient Chemistry Co., Ltd.) and 0.2% of polypeptone (manufactured by Daigo Nutrient Chemistry Co., Ltd.). After the incubation, the cells were harvested by centrifugation and 4 mg of the ADH complex was then obtained in a conventional manner (the method disclosed in Japanese Patent Application Laid-Open No. 63-12278). This complex was subjected to SDS-polyacrylamide gel electrophoresis to separate the protein having a molecular weight of about 72,000 and the protein having a molecular weight of about 44,000. The respective proteins were eluted from the gel in a conventional manner and provided as samples for the following experiment.

Each 0.1 mg of the samples obtained was subcutaneously injected to rabbit together with complete Freund's adjuvant, and 0.1 mg of each sample was further injected after about 2 weeks. One month after the first injection, rabbit blood was withdrawn from the ear and centrifuged. The reactivity of the thus obtained serum with the two proteins was examined, whereby precipitation was noted. Further after SDS-polyacrylamide gel electrophoresis, its specificity was examined by Western blotting, using the cell-free extract of Acetobacter altoacetigenes MH-24 and E. coli JM 109. Reactivity with proteins other than the objective protein was not appreciable but the antibody having high specificity was produced.

### Cloning of gene containing the full length of the structural gene of ADH complex

The Pst I fragment having a size of about 7.0 kilo base containing the structural gene of the protein having a molecular weight of 72,000, which constructed the ADH complex, obtained by the procedures described above and the Sma I fragment having a size of about 4.5 kilo base were ligated at the Pst I site or Sma I site of E. coli vector pUC18, respectively, in a conventional manner. The ligated chimeric plasmid was transformed to E. coli JM 109 in a conventional manner to give transformants carrying the chimeric plasmids. From the transformants, the plasmids were prepared in a conventional manner followed by analysis with restriction enzymes.

By the analysis with restriction enzymes, selection was made for chimeric plasmids in which the Pst I fragment or the Sma I fragment was inserted in such a way that the transcription direction of the lac promoter of E. coli vector pUC18 was the same as the transcription direction of the structural gene of the protein having a molecular weight of about 72,000, which constructed the ADH complex. The transformants carrying these plasmids were cultured at 37°C for 8 hours in LB medium containing 30 µg/ml of ampicillin and 1 mM of isopropyl-β-thiogalactopyranoside (IPTG).
The cells were sonicated, and the resulting homogenate was subjected to SDS-polyacrylamide gel electrophoresis. A molecular weight of the protein specifically reacting with the antibody was determined using an antibody capable of specifically reacting with the two proteins which constructed the ADH complex described above, according to the Western blotting method (Annal. Biochem., 12, 195 (1981)). When detection was made using the antibody to the protein having a molecular weight of about 72,000, the reaction with the protein having a molecular weight of about 72,000 was noted both in the case of carrying the Pst I fragment and in the case of carrying the Sma I fragment. In the transformant carrying vector pUC18 alone which was used for control, no protein capable of reacting with the antibody was detected. By the foregoing, it was confirmed that the structural gene of the protein having a molecular weight of about 72,000 was present on the Pst I fragment and on the Sma I fragment.

On the other hand, detection was made using the antibody to the protein having a molecular weight of about 44,000. In the transformant carrying only vector pUC18 that was used for control, no protein capable of reacting with the antibody was noted. However, in the transformant carrying the plasmid into which the Sma I fragment had been inserted, the reaction with the protein having a molecular weight of about 24,000 was noted. Further in the transformant carrying the plasmid into which the Pst I fragment had been inserted, the reaction with the protein having a molecular weight of about 44,000 was noted. To the contrary, in the cells cultured in liquid medium containing no IPTG, the protein having a molecular weight of about 44,000 and capable of reacting with the antibody was not detected.

These results indicate that the structural gene encoding the protein having a molecular weight of about 44,000, which is cytochrome c, is present on the Pst I fragment and the direction of its transcription is the same as that of the protein having a molecular weight of about 72,000. From the fact that the molecular weight is about 44,000, it is also assumed that the region of the structural gene necessary for encoding this protein would be about 1.2 kilo base. Taking the size of the protein capable of reacting with the antibody into account, it is assumed that the structural gene of cytochrome c having a molecular weight of about 44,000 would be present immediately downstream the structural gene of the protein having a molecular weight of about 72,000 and transcribed and expressed in one unit.

Based on the foregoing results, it was confirmed that the structural genes of the protein having a molecular weight of about 72,000 and the protein having a molecular weight of about 44,000 are present on the gene fragment cleaved with Pst I in the restriction enzyme map shown in Fig. 1.

### EXAMPLE 2

### Transformation of the gene fragment containing the structural gene of ADH complex into acetic acid bacteria host

Chimeric plasmid pADHS1 of the Sma I fragment (about 4.5 kilo base) containing the structural gene of the protein having a molecular weight of about 72,000, which constructed the ADH complex isolated in EXAMPLE 1 was extracted from E. coli ADHS-1 in a conventional manner to give purified DNA. After 1 µg of this DNA was cleaved with Sac I, the cleavage end was rendered blunt with T4 DNA polymerase. On the other hand, plasmid named pTA5001 was prepared from Acetobacter aceti No. 1023 [FERM BP-2287 (FERM P-7122)] according to the method described in Agricultural and Biological Chemistry, 49, 1011 (1985) (pTA5001 is described in Agricultural and Biological Chemistry, 49, 1011 (1985). pTA5001 is a mixture of two plasmids of pTA5001A having a length of 23.5 kilo base and pTA5001B having a length of 23 kilo base.). After 5 µg of this plasmid DNA werecleaved with Xho I, the cleavage end was rendered blunt with T4 DNA polymerase.

The cleaved DNAs of pADHS1 and pTA5001 prepared as described above were ligated with each other using T4 DNA ligase to give the ligation product. Thereafter, the product was transformed in ADH activity-deleted mutant 10-80 according to the method described in Agricultural and Biological Chemistry, 49, 2091 (1985). The transformants were selected in YPG agar medium (3% of glucose, 0.5% of yeast extract, 0.2% of polypeptide, 2% of agar, pH 6.5) containing 50 µg/ml of ampicillin. Plasmids of 10 ampicillin-resistant strains grown in the selection medium were analyzed by a modified method of Agricultural and Biological Chemistry, 49, 2083 (1985). As the result, the size of the plasmids introduced were all about 31 kilo base. Analysis with restriction enzymes reveals that they were all chimeric plasmid of three components: pUC18, The Sma I fragment of 4.5 kilo base containing the structural gene of the protein having a molecular weight of about 72,000 which constructed the ADH complex, and pTA5001. This chimeric plasmid was named pMADHS1.

After pADHPl was cleaved with Sac I as in pADHSl, chimeric plasmid of plasmid pADHP1 isolated in EXAXPLE 1 and pTA5001 was prepared in a manner similar to the case of pADHSl. The chimeric plasmid was transformed into mutant 10-80 of Acetobacter aceti No. 1023 to give the transformant carrying the chimeric plasmid (named pMADHP1).

### Properties of acetic acid bacteria transformant

With respect to the two transformants of mutant 10-80 of Acetobacter aceti No. 1023 obtained above, enzyme activity of ADH was assayed. Firstly, ampicillin was added to YPG liquid medium (medium having a composition obtained by removing agar from YPG agar medium described above) in a concentration of 30 µg/ml followed by shaking-culture at 30°C for 36 hours. After culturing, the cells were harvested, suspended in McIlvaine buffer (pH 6.0) and homogenized with a French press. ADH activity in the supernatant obtained from the homogenate was measured by a method of Agricultural and Biological Chemistry, 49, 2045 (1978). At the same time, aldehyde dehydrogenase (ALDH) activity was also determined by a method of Agricultural and Biological Chemistry, 44, 503 (1980). These results are shown in Table 1.

**Table 1**

| Chimera Plasmid | | Enzyme Activity (U/mg protein) | |
|---|---|---|---|
| Strain | Carried | ADH | ALDH |
| No. 1023 | none | 0.28 | 0.94 |
| 10-80 | none | 0.01 | 0.85 |
| 10-80 | pMADHS1 | 0.01 | 0.90 |
| 10-80 | pMADHP1 | 0.40 | 1.00 |

Mutant 10-80 obtained from Acetobacter aceti No. 1023 is a strain which is specifically deleted of ADH activity. The transformant of this strain carrying plasmid pMADHS1 containing the structural gene alone encoding the protein having a molecular weight of about 72,000 did not show ADH activity yet. On the other hand, in the transformant carrying plasmid pMADHP1 concurrently containing the gene encoding the protein having a molecular weight of about 44,000, restoration of ADH activity was noted. From the results, it is shown that for expression of ADH activity, two proteins having a molecular weight of 72,00 and a molecular weight of 44,000 which construct the ADH complex are required.

It is also noted that the specific activity of the parent having no chimeric plasmid was 0.28 (unit/mg protein), whereas the the specific activity of transformant was 0.40, showing an increase of the activity by about 1.4 times.

As described above, the cell content of ADH having the activity can be increased by transforming acetic acid bacteria with the gene containing the structural gene of ADH complex.

### EXAMPLE 3

### Determination of nucleotide sequence of the structural gene of the protein having a molecular weight of about 44,000 which constructs the ADH complex

The results of EXAMPLE 1 reveal that the structural gene encoding the protein having a molecular weight of about 44,000 is present right downstream of the structural gene encoding the protein having a molecular weight of about 72,000. Therefore, the nucleotide sequence of an about 2.8 kilo base fragment containing the region downstream of the structural gene encoding a protein having a molecular weight of about 72,000 in the insert fragment of pADHP1, restriction enzyme map of which is shown in Fig. 1 (from the left Cla I site to the right BamH I site) was determined by the dideoxy method (Methods in Enzymology, 10, 20, Academic Press, New York, 1983), using M13.

Based on the determined nucleotide sequence, the open-reading frame which could encode the protein having a molecular weight of about 44,000 downstream of the nucleotide sequence shown in Fig. 3 was analyzed and an open-reading frame which could encode a protein of 468 amino acid residues (molecular weight of 49757) composed of 1404 bases starting with translation initiation codon ATG as shown in Fig. 4, was found (the amino acid sequence determined from the nucleotide sequence in Fig. 4 is shown in Fig. 4 below the nucleotide sequence). In order to confirm that the polypeptide having the amino acid sequence shown in Fig. 4 coincides with the protein having a molecular weight of about 44,000 which constitutes the membrane-bound alcohol dehydrogenase complex of the present invention, the protein having a molecular weight of about 44,000 was isolated from the membrane-bound alcohol dehydrogenase complex. The protein was treated with Lysyl endopeptidase, the resulting cleavage product was fractionated and the amino terminal amino acid sequence of the resulting peptide was determined, in a manner similar to EXAMPLE 1. It is confirmed that the same amino acid sequence as that determined is present in the sequence shown in Fig. 4. That is, lysyl endopeptidase was acted on the protein having a molecular weight of about 44,000 isolated in a manner similar to EXAMPLE 1. The resulting cleavage product was fractionated by HPLC in a manner similar to EXAMPLE 1. Among the eluted peptides, the first and fourth peptides were fractionated in the earlier order of elution. Using about 0.1 mg of the fractionated product, the amino acid sequence at the amino terminus was determined in a manner similar to EXAMPLE 1. As the result, the amino terminal amino acid sequence of the peptide firstly eluted was determined to be Asp-Phe-Tyr-Pro-Ala-Pro and the amino terminal amino acid sequence of the peptide fourthly eluted was determined to be Ser-Leu-Ser-Ala-Glu-Glu.

These sequences coincided with the sequence after 169 and with the sequence after 390, from the amino terminus, in the amino acid sequence shown in the lower lines in Fig. 4. It was thus confirmed that the gene having the nucleotide sequence shown in Fig. 4 was the structural gene of the protein having a molecular weight of about 44,000 which constituted the ADH complex.

According to the present invention, the structural gene of the ADH complex produced by a series of microorganisms belonging to the genus Acetobacter represented by Acetobacter altoacetigenes can be cloned and the structural gene can be successfully incorporated into a plasmid. Further by using acetic acid bacteria transformed by the plasmid, efficiency of acetic acid fermentation can be increased. Moreover, the ADH complex can be readily extracted and purified from the acetic aced bacteria and this enzyme can be utilized for quantitative determination of alcohol.

While the invention has been described in detail and with reference to specific embodiments thereof, it is apparent to one skilled in the art that various changes and modifications can be made therein without departing from the spirit and the scope of the present invention.

## Claims (Claims for the following Contracting State(s): DE, GB, FR, IT, NL)

1. A structural gene of the membrane-bound alcohol dehydrogenase complex, said gene being comprised in a fragment of about 7.0 kilo bases derived from a microorganism belonging to the genus Acetobacter and having the restriction enzyme map shown in Figure 1, said complex being composed of a protein having a molecular weight of about 72,000 which has the amino acid sequence shown by Seq ID No. 3, and a protein having a molecular weight of about 44,000 which has the amino acid sequence shown by Seq ID No. 4.

2. The structural gene according to claim 1, wherein said protein having a molecular weight of about 72,000 is encoded by the nucleotide sequence represented by Seq ID No. 1, and the protein having a molecular weight of about 44,000 is encoded by the nucleotide sequence represented by Seq ID No. 2.

3. A plasmid containing a structural gene according to claim 1 or 2.

4. An acetic acid bacterium belonging to the genus Acetobacter or the genus Gluconobacter transformed with a plasmid according to claim 3.

5. A process for the preparation of a membrane-bound alcohol dehydrogenase complex wherein an acetic acid bacterium according to claim 4 is cultivated under suitable conditions.

6. The process according to claim 5, additionally comprising the step of isolating said alcohol dehydrogenase complex.

## Claims (Claims for the following Contracting State(s): ES)

1. A process for preparing a structural gene of the membrane-bound alcohol dehydrogenase complex, said gene being comprised in a fragment of about 7.0 kilo bases derived from a microorganism belonging to the genus Acetobacter and having the restriction enzyme map shown in Figure 1, said complex being composed of a protein having a molecular weight of about 72,000 which has the amino acid sequence shown by SEQ ID NO. 3, and a protein having a molecular weight of about 44,000 which has the amino acid sequence shown by SEQ ID NO. 4, comprising cloning said gene from total DNA of a microorganism belonging to the genus Acetobacter.

2. The process according to claim 1, wherein said protein having a molecular weight of about 72,000 is encoded by the nucleotide sequence represented by SEQ ID NO. 1, and the protein having a molecular weight of about 44,000 is encoded by the nucleotide sequence represented by SEQ ID NO. 2.

3. A process for preparing a plasmid containing a structural gene described in claim 1 or 2, comprising ligating the cleavage product of total DNA of a microorganism belonging to the genus Acetobacter with an appropriate restriction enzyme with the cleavage product with an appropriately restricted vector, transforming an appropriate host and detecting a host carrying the desired gene.

4. A process for preparing an acetic acid bacterium belonging to the genus Acetobacter or the genus Gluconobacter transformed with a plasmid described in claim 3, comprising transforming said bacterium with said plasmid and detecting a bacterium carrying the desired gene.

5. A process for the preparation of a membrane-bound alcohol dehydrogenase complex wherein an acetic acid bacterium prepared according to the process of claim 4 is cultivated under suitable conditions.

6. The process according to claim 5, additionally comprising the step of isolating said alcohol dehydrogenase complex.

## Patentansprüche (Patentansprüche für folgende(n) Vertragsstaat(en): DE, GB, FR, IT, NL)

1. Strukturgen eines membrangebundenen Alkoholdehydrogenasekomplexes, wobei das Gen in einem Fragment von etwa 7 kb enthalten ist, das von einem zur Gattung Acetobacter gehörenden Mikroorganismus stammt und die in der Restriktionskarte von Fig. 1 gezeigten Restriktionsstellen aufweist, wobei der Komplex aus einem Protein mit einem Molekulargewicht von etwa 72 000, das die durch SEQ ID NO. 3 repräsentierte Aminosäuresequenz besitzt, und einem Protein mit einem Molekulargewicht von etwa 44 000 zusammengesetzt ist, das die durch SEQ ID NO. 4 repräsentierte Aminosäuresequenz besitzt.

2. Strukturgen nach Anspruch 1, wobei das Protein mit einem Molekulargewicht von etwa 72 000 von der durch SEQ ID NO. 1 repräsentierten Nucleotidsequenz codiert wird und das Protein mit einem Molekulargewicht von etwa 44 000 von der durch SEQ ID NO. 2 repräsentierten Nucleotidsequenz codiert wird.

3. Plasmid, ein Strukturgen nach Anspruch 1 oder 2 enthaltend.

4. Essigsäurebakterium, das zur Gattung Acetobacter oder Gluconobacter gehört, transformiert mit einem Plasmid nach Anspruch 3.

5. Verfahren zur Herstellung eines membrangebundenen Alkoholdehydrogenasekomplexes, wobei ein Essigsäurebakterium nach Anspruch 4 unter geeigneten Bedingungen gezüchtet wird.

6. Verfahren nach Anspruch 5, außerdem den Schritt der Isolierung des Alkoholdehydrogenasekomplexes umfassend.

## Patentansprüche (Patentansprüche für folgende(n) Vertragsstaat(en): ES)

1. Verfahren zur Herstellung eines Strukturgens eines membrangebundenen Alkoholdehydrogenasekomplexes, wobei das Gen in einem Fragment von etwa 7 kb enthalten ist, das von einem zur Gattung Acetobacter gehörenden Mikroorganismus stammt und die in der Restriktionskarte von Fig. 1 gezeigten Restriktionsstellen aufweist, wobei der Komplex aus einem Protein mit einem Molekulargewicht von etwa 72 000, das die durch SEQ ID NO. 3 repräsentierte Aminosäuresequenz besitzt, und einem Protein mit einem Molekulargewicht von etwa 44 000 zusammengesetzt ist, das die durch SEQ ID NO. 4 repräsentierte Aminosäuresequenz besitzt, wobei das Verfahren die Clonierung des Gens aus Gesamt-DNA eines zur Gattung Acetobacter gehörenden Mikroorganismus umfaßt.

2. Verfahren nach Anspruch 1, wobei das Protein mit einem Molekulargewicht von etwa 72 000 von der durch SEQ ID NO. 1 repräsentierten Nucleotidsequenz codiert wird und das Protein mit einem Molekulargewicht von etwa 44 000 von der durch SEQ ID NO. 2 repräsentierten Nucleotidsequenz codiert wird.

3. Verfahren zur Herstellung eines ein in den Ansprüchen 1 oder 2 beschriebenes Strukturgen enthaltenden Plasmids, umfassend die Ligierung des Spaltprodukts von Gesamt-DNA eines zur Gattung Acetobacter gehörenden Mikroorganismus, die mit einem geeigneten Restriktionsenzym gespalten wurde, mit einem geeignet gespaltenen Vektor, die Transformation eines geeigneten Wirts und den Nachweis eines das gewünschte Gen tragenden Wirts.

4. Verfahren zur Herstellung eines zur Gattung Acetobacter oder Gluconobacter gehörenden, mit einem in Anspruch 3 beschriebenen Plasmid transformierten Essigsäurebakteriums, umfassend die Transformation des Bakteriums mit dem Plasmid und den Nachweis eines das gewünschte Gen tragenden Bakteriums.

5. Verfahren zur Herstellung eines membrangebundenen Alkoholdehydrogenasekomplexes, wobei ein gemäß dem Verfahren nach Anspruch 4 hergestelltes Essigsäurebakterium unter geeigneten Bedingungen gezüchtet wird.

6. Verfahren nach Anspruch 5, außerdem den Schritt der Isolierung des Alkoholdehydrogenasekomplexes umfassend.

## Revendications (Revendications pour l'(les) Etat(s) contractant(s) suivant(s): DE, GB, FR, IT, NL)

1. Gène de structure du complexe alcool déshydrogénase lié à la membrane, ledit gène étant inclus dans un fragment d'environ 7.0 kilo base provenant d'un microorganisme appartenant au genre Acétobacter et ayant une carte de restriction enzymatique présentée à la Figure 1, ledit complexe étant composé d'une protéine de poids moléculaire d'environ 72000 dont la séquence d'acides aminés est donnée par Seq ID No. 3, et d'une protéine de poids moléculaire d'environ 44000 dont la séquence d'acides aminés est donnée par Seq ID No. 4.

2. Gène de structure selon la revendication 1, dans laquelle la dite protéine de poids moléculaire d'environ 72000 est codée par la séquence nucléotidique représentée par Seq ID No. 1, et la protéine de poids moléculaire d'environ 44000 est codée par la séquence nucléotidique représentée par Seq ID No. 2.

3. Plasmide contenant le gène de structure selon les revendications 1 et 2.

4. Bactérie à acide acétique appartenant au genre Acétobacter ou au genre Gluconobacter transformée par un plasmide selon la revendication 3.

5. Procédé pour la préparation du complexe alcool déshydrogénase lié à la membrane dans lequel une bactérie à acide acétique selon la revendication 4 est cultivée dans des conditions adéquates.

6. Procédé selon la revendication 5, comprenant en plus l'étape d'isolement dudit complexe alcool déshydrogénase.

## Revendications (Revendications pour l'(les) Etat(s) contractant(s) suivant(s): ES)

1. Procédé pour la préparation du gène de structure du complexe alcool déshydrogénase lié à la membrane, ledit gène étant inclus dans un fragment d'environ 7.0 kilo base provenant d'un microorganisme appartenant au genre Acétobacter et ayant une carte de restriction enzymatique présentée à la Figure 1, ledit complexe étant composé d'une protéine de poids moléculaire d'environ 72000 dont la séquence d'acides aminés est donnée par Seq ID No. 3, et d'une protéine de poids moléculaire d'environ 44000 dont la séquence d'acides aminés est donnée par Seq ID No. 4, comprenant le clonage dudit gène à partir de l'ADN total d'un microorganisme appartenant au genre Acétobacter.

2. Procédé selon la revendication 1, dans laquelle la dite protéine de poids moléculaire d'environ 72000 est codée par la séquence nucléotidique représentée par Seq ID No. 1, et la protéine de poids moléculaire d'environ 44000 est codée par la séquence nucléotidique représentée par Seq ID No. 2.

3. Procédé pour la préparation d'un plasmide contenant un gène de structure décrit dans les revendications 1 et 2, comprenant la liaison du produit de clivage de l'ADN total d'un microorganisme appartenant au genre Acétobacter coupé avec une enzyme de restriction appropriée avec le produit de clivage d'un vecteur approprié, transformant un hôte approprié et sélectionnant un hôte porteur du gène désiré.

4. Procédé pour la préparation d'une bactérie à acide acétique appartenant au genre Acétobacter ou au genre Gluconobacter transformée par un plasmide selon la revendication 3, comprenant la transformation de ladite bactérie avec ledit plasmide et la détection d'une bactérie porteuse du gène désiré.

5. Procédé pour la préparation du complexe alcool déshydrogénase lié à la membrane dans lequel une bactérie à acide acétique préparée selon la revendication 4 est cultivée dans des conditions adéquates.

6. Procédé selon la revendication 5, comprenant en plus l'étape d'isolement dudit complexe alcool déshydrogénase.
